# EUROPEAN PATENT APPLICATION

(11) **EP 2 362 216 A1**
(43) Date of publication of application: **31.08.2011**
(21) Application number: 10002036.1
(22) Date of filing: 27.02.2010
(51) Int. Cl.: G01N 33/00, G01N 27/12, G01N 27/414

(54) **Carbon nanotube SB-FET hydrogen sensor and methods for its manufacture and operation**

(71) Applicant: Karlsruher Institut für Technologie, 76131 Karlsruhe (DE)
(72) Inventor: Fichtner, Maximilian, 68723 Oftersheim (DE); Krupke, Ralph, 76297 Stutensee (DE); von Löhneysen, Hilbert, 76185 Karlsruhe (DE); Hennrich, Frank, 76187 Karlsruhe (DE); Ganzhorn, Marc, 38000 Grenoble (FR); Vijayaraghavan, Aravind, Manchester M3 4NB (GB); Dehm, Simone, 75045 Walzbachtal (DE); Kappes, Manfred, 76275 Ettlingen (DE)

(57) **Abstract**

The present invention relates to a hydrogen sensor which comprises a Schottky-barrier field-effect transistor (SB-FET) with a source electrode (1) and a drain electrode (2), where the source electrode (1) and/or the drain electrode (2) incorporate metallic palladium and where both electrodes are separated from a gate electrode (3) by a dielectric (4). A film (5), which comprises a plurality of semiconducting carbon nanotubes, is embedded between the source electrode (1) and the drain electrode (2) in such a way that it is separated from the gate electrode (3) by the dielectric (4).

The operation of this device is as follows: A gate voltage V_{G} is applied to the gate electrode (3), and a source-drain voltage V_{SD} is applied between the source electrode (1) and the drain electrode (2), resulting in a source-drain current I_{SD}. By monitoring V_{SD} and I_{SD} and determining the conductance G = I_{SD}/V_{SD}, the amount of hydrogen in an atmosphere in contact with the film (5) can be derived from the change of the conductance G upon contact with molecular hydrogen.

## Description

The present invention relates to the field of nanotechnology, in particular to a hydrogen sensor, a method for manufacturing said sensor, and a method for operating said sensor.

The application of hydrogen as an energy carrier in a hydrogen economy is expected to generate a considerable demand of hydrogen sensors which are sensitive, reliable, small, and cost-effective. Known hydrogen sensors are based on electrochemical response, mass spectrometry, measurement of thermal conductivity, and measurement of electrical resistance.

For mass applications, sensors based on resistance changes are most attractive since they are easy to miniaturize and to integrate into read-out electronics. Their central sensing unit is either a field-effect transistor (FET) with a hydrogen-sensitive (catalytic) gate electrode, or a hydrogen sensitive material.

A well-studied example for a hydrogen sensitive material is palladium. F. Yang, D.K. Taggart, and R.M. Penner, Fast, Sensitive Hydrogen Gas Detection Using Single Palladium Nanowires That Resist Fracture, Nano Lett. 9 (2009) 2177, have shown that palladium changes its volume and resistance under hydrogen loading.

Recently, palladium has recieved a lot of attention by the application of Pd nanoparticles with a large surface to volume ratio yielding very short response time. F. Favier, E.C. Walter, M.P. Zach, Th. Benter, and R.M. Penner, Hydrogen Sensors and Switches from Electrodeposited Palladium Mesowire Arrays, Science 293 (2001) 2227, fabricated hydrogen sensors and hydrogen-activated switches from arrays of mesoscopic palladium wires. However, at low hydrogen partial pressure the observed changes were small, thus imposing a fundamental limitation of this sensing principle, besides stress induced failure upon repeated cycling.

An alternative to the measurements of bulk properties is a surface sensitive technique, which is able to detect changes of the Pd surface caused by atomic hydrogen.

H. Conrad, G. Ertl, and E.E. Latta, Adsorption of hydrogen on palladium single crystal surfaces, Surface Science 41 (1974) 435, have demonstrated that a dipole layer of hydrogen is formed at low H₂ partial pressure by the dissociation of the molecular hydrogen and increases the work function of Pd.

R. Dus, R. Nowakowski, and E. Nowicka, Chemical and structural components of work function changes in the process of palladium hydride formation within thin Pd film, J. of Alloys and Compounds 404-406 (2005) 284, describe that at higher H₂ partial pressure Pd-hydride forms additionally reduces the Pdwork function,.

J. Svensson, A.A. Sourab, Y. Tarakanov, D.S. Lee, S.J. Park, S. J. Baek, Y.W. Park, and E.E.B. Campbell, The dependence of the Schottky barrier height on carbon nanotube diameter for Pd-carbon nanotube contacts, Nanotechnology 20 (2009) 175204, have shown that a Schottky barrier, which forms at the interface between a metal and a semiconductor, is a useful sensing element since the electric current through a Schottky barrier depends exponentially on the work functions of the two adjoining materials. For macroscopic Schottky barriers, however, hydrogen must diffuse either through the palladium or the semiconductor layer to reach the barrier, which very likely causes detrimental effects on the response dynamics.

S. Heinze, J. Tersoff, R. Martel, V. Derycke, J. Appenzeller, and Ph. Avouris, Carbon Nanotubes as Schottky Barrier Transistors, Phys. Rev. Lett. 89 (2002) 106801, demonstrate that a nanoscale transistor based on semiconducting single-walled carbon nanotubes (sem-SWNTs) operates as a Schottky-barrier field-effect transistor (SB-FET), where the transconductance is only limited by the transmission of the barrier and not by the conductance of the channel.

A. Javey, J. Guo, Q. Wang, D. Mann, M. Lundstrom, and H. Dai, Ballistic carbon nanotube field-effect transistors, Nature 424 (2003) 654, have demonstrated that SB-FETs formed by sem-SWNTs and palladium source and drain electrodes are sensitive to hydrogen. However, the hydrogen sensitivity of a nanotube-based SB-FET and Pd-coated nanotube device is generally rather low.

F. Hennrich, S. Lebedkin, and M.M. Kappes, Improving separation techniques for single-walled carbon nanotubes: Towards monodisperse samples, Phys. stat. sol. (b) 245 (2008) 1951, describe polymer wrapping (POL) as a method for the facile production of monochiral single-walled carbon nanotube suspensions, where density gradient centrifugation or selective dispersion with special organic polymers is applied.

A. Vijayaraghavan, S. Blatt, D. Weissenberger, M. Oron-Carl, F. Hennrich, D. Gerthsen, H. Hahn, and R. Krupke, Ultra-Large-Scale Directed Assembly of Single-Walled Carbon Nanotube Devices, Nano Letters 7 (2007) 1556, describe dielectrophoretic (DEL) assembling. It is shown that the dielectrophoretic force fields change incisively as nanotubes assemble into the contact areas, leading to a reproducible directed assembly which is self-limiting in forming single-tube devices.

The main object of the present invention is therefore to provide a hydrogen sensor, a method for manufacturing said sensor, and a method for operating said sensor, which overcome the limitations known from the state of the art.

In particular, it is an object of the present invention to provide a small-sized hydrogen sensor with a high and reliable sensitivity to molecular hydrogen.

Another object of the invention is to provide a facile method of manufacturing said sensor.

A further object of the invention is to provide a reliable method of operating said sensor, particularly over a long period of time.

The solution of this problem is provided by a hydrogen sensor according to claim 1, a method of manufacturing said sensor according to claim 7, and a method of operating said sensor according to claim 12. The dependent claims describe preferred features.

According to the present invention, a hydrogen sensor based on a Schottky-barrier field-effect transistor (SB-FET) is provided, which exhibits a source electrode, a drain electrode and a dielectric which separates the source electrode and the drain electrode from a gate electrode. Hereby, the source electrode and the drain electrode consist of a metallic or semiconducting material or possess at least a cover of such a material on their surface, where at least one of said electrodes consists of metallic palladium or possesses a cover of said material. Preferably, the source electrode and the drain electrode are arranged on an insulating substrate.

According to the invention, a film, which includes of a plurality of semiconducting carbon nanotubes, is embedded between the source electrode and the drain electrode in such a way that said film is separated by the dielectric from the gate electrode.

In a specific embodiment, said film includes either single-walled carbon nanotubes or multi-walled carbon nanotubes, of which all walls are semiconducting, or a mixture thereof.

In a preferred embodiment, said film predominantly consists of a plurality of semiconducting carbon nanotubes with a diameter from 0.8 nm to 5.0 nm, in particular with a diameter from 1 nm to 1.4 nm.

In a particularly preferred embodiment, said film predominantly consists of a plurality of monochiral semiconducting carbon nanotubes with a chirality of (9,7).

In a further preferred embodiment the gate electrode is chargeable by a gate voltage V_{G} with alternating plus or minus signs. This feature specifically increases the long-term sensitivity of the sensor.

Another aspect of the present invention is directed to a method of manufacturing said hydrogen sensor according to the following steps.

An insulating substrate is provided on which a source electrode and a drain electrode are arranged. Suitable substrates are made of insulating or semi-conducting organic or inorganic materials, e.g. Si/SiO₂ or polymers films like parylene.

Further, a plurality of carbon nanotubes is provided and sorted according to their electronic properties and/or diameters into at least two fractions. Preferred methods for sorting are density gradient centrifugation or selective dispersion. The sorting is performed in such a way that at least one fraction is obtained which predominantly consists of semiconducting carbon nanotubes.

Such single or multiple fractions which predominantly consist of semiconducting carbon nanotubes are selected. An amount thereof is deposited as a film between the source electrode and the drain electrode. A preferred method for depositing is dielectrophoretic assembling.

Further, a gate electrode is arranged in such a way that it is separated from said film, which includes semiconducting carbon nanotubes, by a dielectric. Hereto either said insulating substrate is applied as dielectric or a separate dielectric layer is provided.

Also, means for electrically connecting said electrodes are provided.

In a preferred embodiment, said plurality of carbon nanotubes is processed within a dispersion, which particularly includes a non-polar organic solvent, e.g. toluene.

Another aspect of the present invention is directed to a method of operating said hydrogen sensor according to the following steps.

A gate voltage V_{G} is applied to the gate electrode.

A source-drain voltage V_{SD} is applied between the source electrode and the drain electrode, which results in a source-drain current I_{SD.}

The source-drain voltage V_{SD} and the source-drain current I_{SD} are monitored, and the value of the conductance G = I_{SD}/V_{SD} is determined.

When said film, which includes a plurality of semiconducting carbon nanotubes, is brought into contact with molecular hydrogen, a change in the value of the conductance G is observed.

Using a previously calibrated sensor will allow to directly correlate the value of the conductance G with the percentage of molecular hydrogen in the atmosphere surrounding the sensor.

In a particularly preferred embodiment, the gate voltage V_{G} is applied with alternating plus or minus signs over time. Preferably, the time constant for said alternation is in the range between 1 ns and 10 s, in particular between 1 ms and 10 s. Hereby, the upper limit of the time constant is determined by the characteristic time constant for charge migration in such a system whereas the lower limit of the time constant is only determined by the capacity of the device. This way of operation increases the long-term sensitivity of the sensor.

A hydrogen sensor according to the present invention provides several advantages. It is operable in ambient air and temperature and works both on organic or inorganic isolating substrates. Also, its fabrication is straightforward.

The present invention will be more apparent from the following description of non-limiting specific embodiments with reference to the drawings.
**Fig. 1a) to Fig. 1c****)** schematically illustrate two different set-ups of a hydrogen sensor according to the present invention. In **Fig. 1d****)** a model describing the functionality of the invention is presented.
**Fig. 2** displays the source drain currents I_{SD} versus the gate voltages V_{G} for **a)** a (9,7)/Pd-SB-FET, **b)** a (7,5)/Pd-SB-FET and **c)** a (6,5)/Pd-SB-FET device, each on a Si/SiO₂ substrate and under flow of air and 100 ppm H₂ in Ar, respectively.
**Fig. 3** exhibits the time dependence of the response of the *On-state conductance* G_{SD} which follows the switching of the atmosphere surrounding the sensor from air via H₂/air to air for **a)** a (9,7) /Pd-SB-FET, **b)** a (7,5) /Pd-SB-FET, and **c)** a (6,5)/Pd-SB-FET device, each on a Si/SiO₂ substrate.
**Fig. 4** shows for a (9,7)/Pd-SB-FET device on Parylene/Si/SiO₂ substrate **a)** the source drain currents I_{SD} versus the gate voltages V_{G}, and **b)** the time dependence of the *On-state conductance* G_{SD} which follows the switching of the atmosphere surrounding the sensor from air via H₂/air to air.
**Fig. 5** displays an analysis of various *On-state conductance* measurements in air and in H₂, G_{ON,air}/G_{ON,H2} versus the corresponding nanotube diameter d.
**Fig. 6** shows the source drain currents I_{SD} versus time of a (9,7)/Pd-SB-FET device on Si/SiO₂ substrate with **a)** under constant gain voltage V_{G}, and **b)** under the indicated alternating gain voltage V_{G} variation over time.

### Methods and materials

Schottky-barrier field-effect transistors (SB-FETs) were produced by combining chirality selective polymer wrapping (POL) of SWNTs with dielectrophoretic (DEP) assembling from dispersion. For this purpose, the following toluene dispersions with sem-SWNTs of different chiralities (n,m) were prepared:
- A first dispersion, produced from pulsed laser vaporization material, containing > 90 % (9,7) SWNT with a diameter d = 1.1 nm;
- A second dispersion, produced from HiPCO material, containing 50 % (7,5) and 25 % (7,6) SWNT with diameters d = 0.83 nm and 0.895 nm, respectively;
- A third dispersion, produced from HiPCO material, containing > 90 % (7,6) SWNT with a diameter d = 0.895 nm,; and
- A forth dispersion, produced from CoMoCat material, containing 75 % (6,5) SWNT with a diameter d = 0.757 nm.

The toluene dispersions containing the sem-SWNTs were deposited by dielectrophoresis between Pd(60 nm)/Ti(3 nm) or Au(60 nm)/Ti(3 nm) electrodes on thermally oxidized (200 nm or 800 nm) p-doped (100) Si with < 0.001 Ω•cm. The electrodes, with gaps of 300 nm, were defined by electron beam lithography and metal sputtering.

Additional SB-FETs were prepared on substrates coated with 100 nm Parylene-C, i. e. a hydrophobic organic polymer layer, deposited by a chemical vapor deposition technique.

The SB-FETs were Au- or Al-wire bonded into a ceramic package for device characterization and operation.

All devices are labeled by the SWNT majority species of the corresponding dispersion. In total, 3x (9,7)/Pd-SB-FET, 1x (7,5)/Pd-SB-FET, 3x (6,5)/Pd-SB-FET, 2x (9,7)/Au-SB-FET, 2x (7,6)/Au-SB-FET, 2x (6,5)/Au-SB-FET devices on SiO2/Si substrates, and 3x (9,7)/Pd-SB-FET, 3x (6,5)/Pd-SB-FET devices on Parylene/SiO2/Si substrates were prepared.

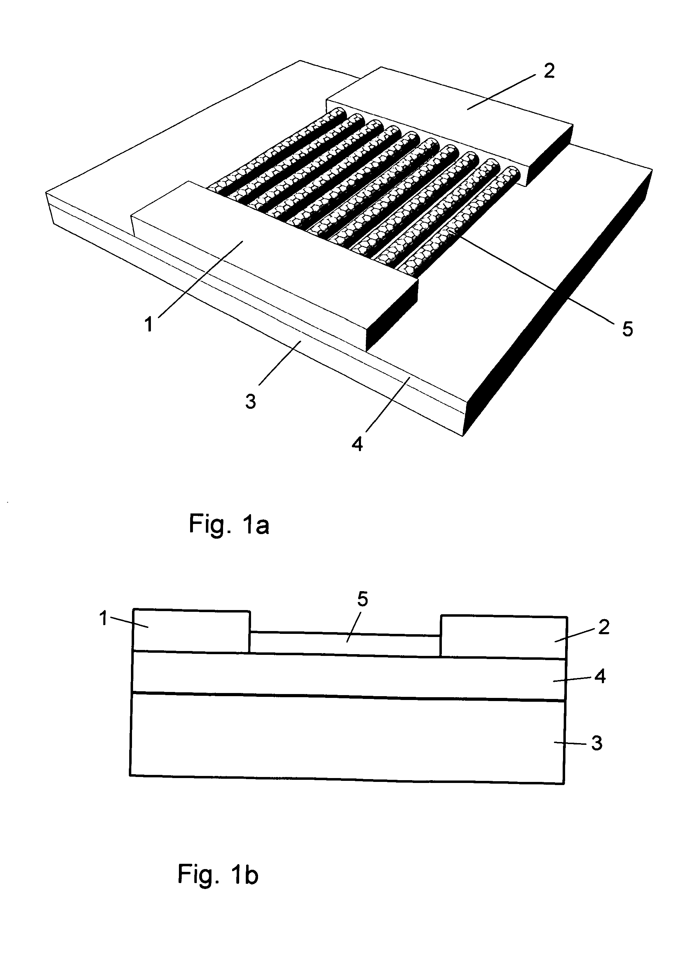

In **Fig. 1a****) and Fig. b)** the set-up of the hydrogen sensor is schematically illustrated in **a)** an aerial view and **b)** in a side view, respectively. A palladium source electrode **1** and a palladium drain electrode **2** are arranged on an isolating silicon (Si) or parylene substrate which acts as a dielectric **4**. Said isolating layer is based itself on a silicon dioxide (SiO₂) layer acting as a gate electrode **3**. Between the source electrode **1** and the drain electrode **2** a film **5** which comprises a plurality of semiconducting carbon nanotubes is arranged on top of said isolating substrate.

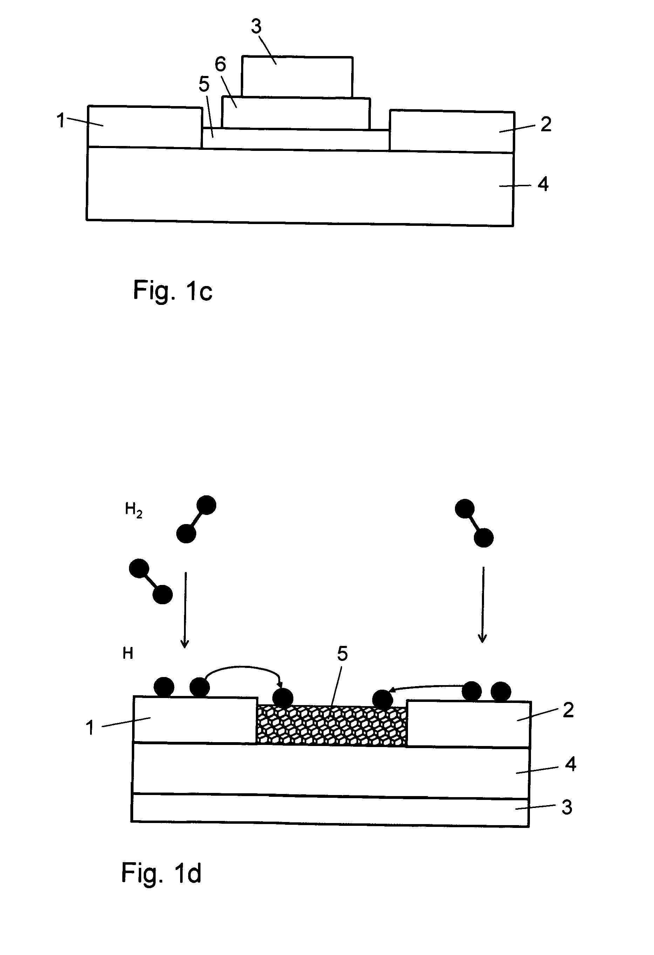

**Fig. 1c****)** schematically shows a different set-up of the hydrogen sensor in a side view. A palladium source electrode **1** and a palladium drain electrode **2** are arranged such on an insulating silicon (Si) or parylene substrate that a film **5** which comprises a plurality of semiconducting carbon nanotubes is embedded between them on top of said isolating substrate. On top of said film **5** a separate dielectric **6** is arranged in such a way that it separates said film **5** from a gate electrode **3**.

**Fig. 1d****)** presents a model which describes the functionality of the invention. Here, the only source of atomic hydrogen H is the surface of the palladium source electrode **1** and palladium drain electrode **2**, where H₂ has been catalytically dissociated. H diffuses from the palladium surface onto the SWNTs in order to bind onto medium diameter SWNTs.

The SB-FETs were introduced in a controlled atmosphere in a cavity with a volume of approx. 1 cm³, which was connected to a pipe system. Copper was used as the lower half cavity material and pipes from stainless steel to prevent hydrogen poisoning of the system. The ceramic package with the SB-FETs formed the upper half of the cavity and was mounted upside down in order to close the cavity with an airtight O-ring sealing. The cavity was connected to valve system which allowed the switching between different gas flows, in particular air, Ar, or H₂ in Ar. All measurements were carried out at flow rates of 0.5 L/min, corresponding to a dynamic pressure of 1 to 10 Pa and a static gas pressure of p = 0,1 MPa. The gas in the cavity nominally exchanged within 0.1 s, thus setting the time resolution. All results were obtained at ambient temperatures of approx. 300 K.

### Experimental results

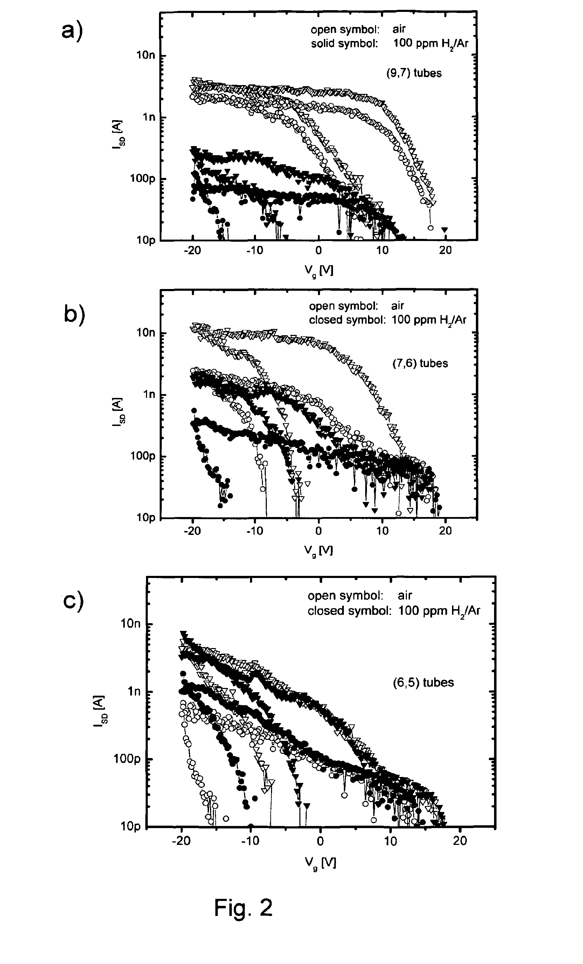

In **Fig. 2** the characteristics of (9,7)/Pd-SB-FET, (7,5)/Pd-SB-FET and (6,5)/Pd-SB-FET devices on Si/SiO₂ substrates under flow of air and 100 ppm H₂ in Ar are compared. All devices show enhanced p-type conductance under negative gate bias with a pronounced hysteresis, which is typical for sem-SWNTs in contact with Pd.

The *On-state conductance* G_{ON} = I_{SD,ON}/V_{SD,ON} is reached for all devices around V_{G} = -20 V, with a weak dependence on V_{SD} with the source drain voltage V_{SD}, the source drain current I_{SD}, and the gate voltage V_{G}, with respect to the grounded drain electrode.

G_{ON} is reduced under H₂ exposure, where the sensitivity strongly depends on the chirality of the nanotube (n,m). Whereas the current is suppressed by a factor of 10 for (9,7)/Pd-SB-FET devices and a factor of 5 for (7,5)/Pd-SB-FET devices, it remains unchanged for (6,5)/Pd-SB-FET devices. The data in H₂/Ar have been acquired 3 minutes after switching from air to 100 ppm H₂ in Ar.

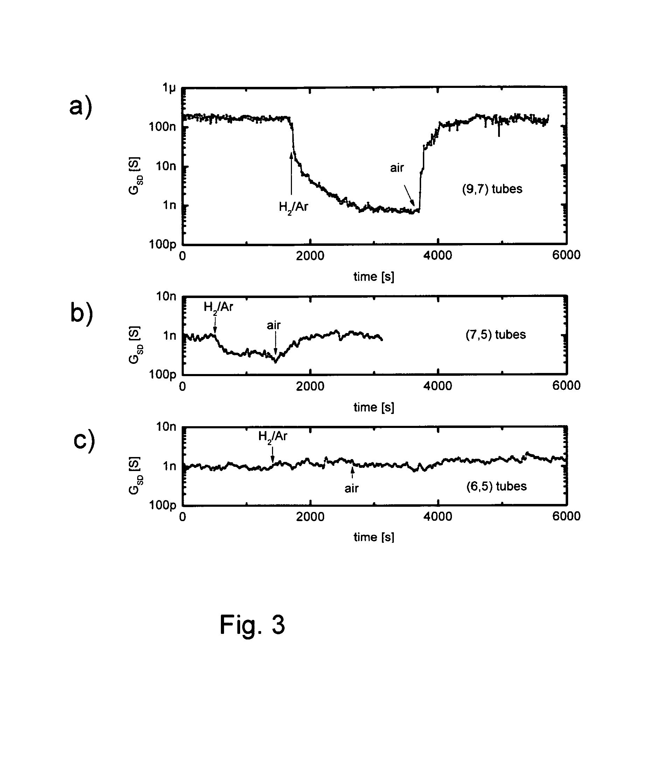

**Fig. 3** shows the time dependence of the response of the On-*state conductance* G_{SD} on the switching of the gases from air via H_{2/}air to air for **a)** a (9,7)/Pd-SB-FET device, **b)** a (7,5)/ Pd-SB-FET device, and **c)** a (6,5)/Pd-SB-FET device, each on a Si/SiO₂ substrate.

The response for the (9,7)/Pd-SB-FET device is characterized by two time scales: An initial fast response, where G_{SD} changes with a rate of 30 s per current decade, and a slow response, where the steady-state I_{SD} is reached on the timescale of hours. The change of I_{SD} within the fast response is on the order of one and two decades for the switching from air to H₂/air and from H₂/air to air, respectively. In the steady state, G_{SD} for 100 ppm H₂ in Ar is suppressed by a factor of 500 compared to I_{SD} in air.

The response times is much slower and the sensitivity much weaker for a (7,5)/Pd-SB-FET device.

No time dependence could be measured for a (6,5)/Pd-SB-FET device.

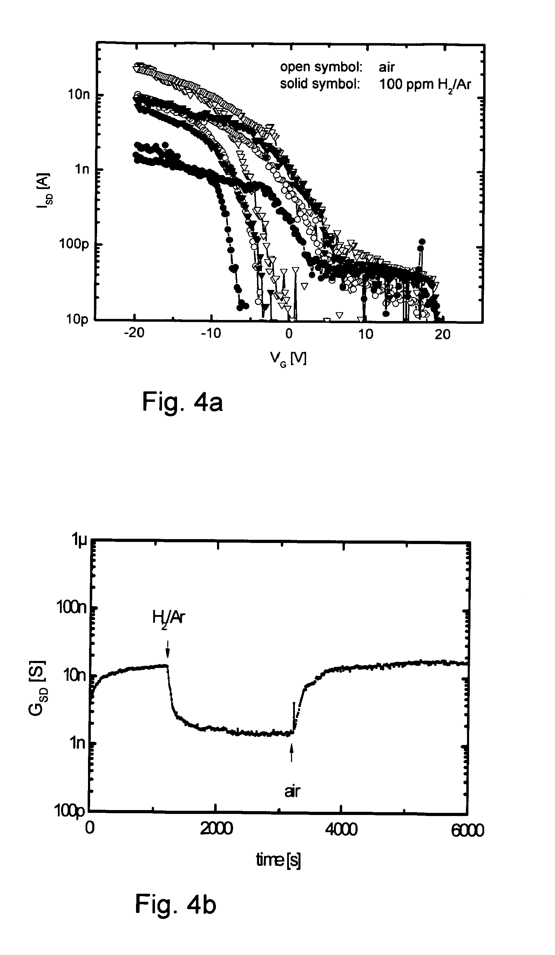

**Fig. 4** demonstrates that a similar chirality dependence with devices prepared on a substrate of Parylene-C/Si/SiO₂, albeit with lower sensitivity, was observed.

### Structural explanation

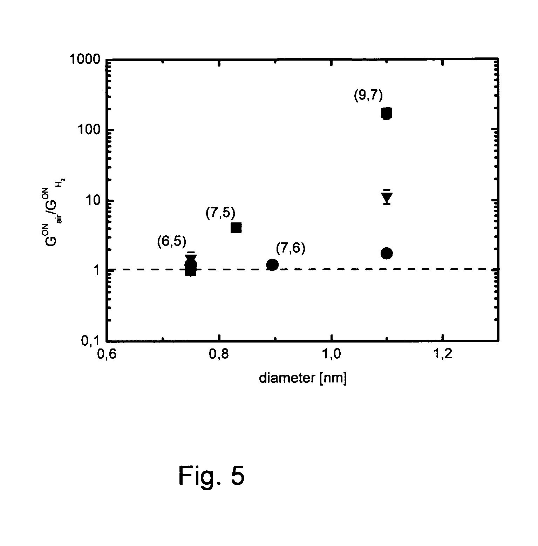

**Fig. 5** exhibits a plot of the ratio of the *On-state conductance* measured in air and in H₂, G_{ON},_{air/}G_{ON},_{H2} versus the nanotube diameter d, which shows that the sensitivity to H₂ increases with increasing d. This plot demonstrates that the sensitivity to hydrogen of SB-FET from (n,m) sorted semiconducting SWNT in contact with Pd electrodes depends on the nanotube diameter.

The overall sensitivity of a sem-SWNT-based SB-FET to hydrogen has been reported before by A. Javey et al., who assigned this feature to changes in the workfunction of Pd. However, in contrast to their findings, it is a characteristic of the present invention that the sensitivity to hydrogen depends on the electronic properties of the SWNT, in particular their chirality (n,m).

This feature is difficult to understand solely on changes of workfunction of Pd, and hence requires a consideration of the interaction of hydrogen with SWNTs. Taking into account that the chiral angle θ of the different SWNTs species is very similar, i.e. θ = 25.9 to 27.5° for a SWNT with a (9,7), a (7,5) and a (6,5) chirality, the observed chirality dependence is rather a dependence on the nanotube diameter d.

Therefore, the high sensitivity for devices from medium diameter (9,7) SWNT can be explained by a decrease of Pd work function by chemisorbed atomic hydrogen. The decreased or diminished sensitivity for small diameter (7,5) and (6,5) chiralities, however, can only be explained if an additional change of the nanotube work function is taken into account. Such a change is rationalized by chemisorption of atomic hydrogen to the nanotube sidewall, which spills over from the Pd surface.

The diameter dependence shows that it is important to use sorted carbon nanotubes for sensor fabrication and explains why sensors based on ensembles of unsorted nanotubes have a low sensitivity. So far (9,7)/Pd SB-FETs show the highest sensitivity with up to 2-3 orders of magnitude current suppression at 100 ppm H₂.

### Long-term stability

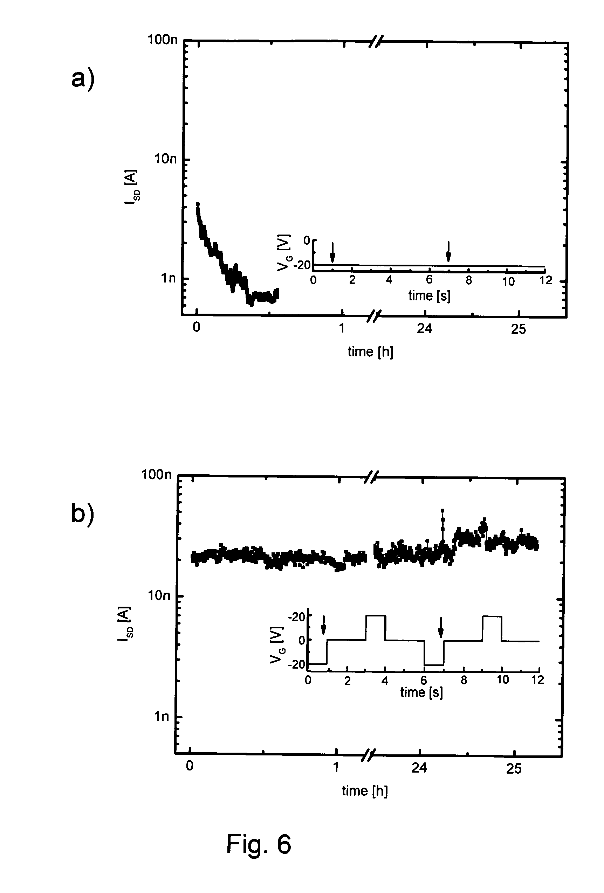

Long-term stability is a relevant parameter for a sensor application. **Fig. 6a****)** shows that a substantial reduction of the sensitivity of the SWNT/Pd-FETs occurs within an hour of measurement at constant gate voltage V_{G} = 20 V together with an overall reduction of source drain current I_{SD} independent of the atmosphere Ar, air or H_{2/}Ar.

**Fig. 6b****)** demonstrates that a considerably increased long-term stability is achieved by inverting the gate voltage periodically. A time constant in the range of 1-2 s has been chosen for the alternation. It is assumed that the aging is due to screening of the gate field by charges which propagate from the SWNT into the substrate under constant gate voltage. Thereby, the gate voltage decreases effectively which, as a result, reduces the source drain current. The application of an alternating gate voltage inhibits an accumulation of space charge and thus ensures long term stability. It is mentioned that all time-dependent data have been acquired with an alternating gate voltage.

## Claims

1. Hydrogen sensor, comprising a Schottky-barrier field-effect transistor (SB-FET) with a source electrode (1) and a drain electrode (2), where the source electrode (1) and/or the drain electrode (2) incorporate metallic palladium and where both electrodes are separated from a gate electrode (3) by a dielectric (4), **characterized in that** a film (5), which comprises a plurality of semiconducting carbon nanotubes, is embedded between the source electrode (1) and the drain electrode (2) in such a way that it is separated from the gate electrode (3) by the dielectric (4).

2. Hydrogen sensor according to claim 1, **characterized in that** said film (5) comprises a plurality of single-walled carbon nanotubes or multi-walled carbon nanotubes where all walls are semiconducting.

3. Hydrogen sensor according to claim 1 or 2, **characterized in that** said film (5) predominantly comprises a plurality of semiconducting carbon nanotubes with a diameter from 0.8 nm to 5.0 nm.

4. Hydrogen sensor according to claim 3, **characterized in that** said film (5) predominantly comprises a plurality of semiconducting carbon nanotubes with a diameter from 1 nm to 1.4 nm.

5. Hydrogen sensor according to claim 4, **characterized in that** said film (5) predominantly comprises a plurality of monochiral semiconducting carbon nanotubes with a chirality of (9,7).

6. Hydrogen sensor according to one of claims 1 to 5, **characterized in that** the gate electrode (3) is chargeable by a gate voltage V_{G} with alternating plus or minus signs.

7. Method of manufacturing a hydrogen sensor according to one of claims 1 to 6, such method having the following steps:
- providing an insulating substrate and superimposing on said insulating substrate a source electrode (1) and a drain electrode (2),
- further providing a plurality of carbon nanotubes and sorting said carbon nanotubes according to their electronic properties into at least two fractions, where at least one of said fraction predominantly comprises semi-conducting carbon nanotubes,
- selecting at least one of said fractions which predominantly comprises semiconducting carbon nanotubes, and depositing an amount thereof as film (5) on said insulating substrate between the source electrode (1) and the drain electrode (2),
- arranging a gate electrode (3) such that it is separated from said film (5) by a dielectric (4, 6), and
- providing means for electrically connecting said source electrode (1), said drain electrode (2), and said gate electrode (3).

8. Method according to claim 7, **characterized in that** the sorting of said carbon nanotubes into at least two fractions is achieved via density gradient centrifugation or selective dispersion.

9. Method according to claim 7 or 8, **characterized in that** the depositing of said fraction of carbon nanotubes between the source electrode (1) and the drain electrode (2) is achieved via dielectrophoretic assembling.

10. Method according to one of claims 7 to 9, **characterized in that** said plurality of carbon nanotubes is processed within a dispersion.

11. Method according to claim 10, **characterized in that** said dispersion comprises a non-polar organic solvent.

12. Method of operating a hydrogen sensor according to one of claims 1 to 6, such method having the following steps:
- applying a gate voltage V_{G} to the gate electrode (3),
- applying a source-drain voltage V_{SD} between the source electrode (1) and the drain electrode (2), resulting in a source-drain current I_{SD},
- monitoring the source-drain voltage V_{SD} and the source-drain current I_{SD}, and determining the value of the conductance G = I_{SD}/V_{SD},
- bringing the film (5), which comprises a plurality of semiconducting carbon nanotubes, into contact with molecular hydrogen, thus observing a change of the value of the conductance G upon contact with molecular hydrogen.

13. Method according to claim 12, **characterized in that** the gate voltage V_{G} is applied with alternating plus or minus signs over time.

14. Method according to claim 12 or 13, **characterized in that** the time constant for said alternation of the gate voltage V_{G} is in the range between 1 ms and 10 s.
